# EUROPEAN PATENT APPLICATION

(11) **EP 4 696 229 A1**
(43) Date of publication of application: **18.02.2026**
(21) Application number: 24194953.6
(22) Date of filing: 16.08.2024
(51) Int. Cl.: A61B 5/024, A61B 5/055, A61B 5/00, G01R 33/32, G01R 33/567

(54) **HEAD COIL DEVICE WITH LIGHT SOURCE FOR REMOTE PHOTOPLETHYSMOGRAPHY**

(71) Applicant: Koninklijke Philips N.V., 5656 AG Eindhoven (NL)
(72) Inventor: WEISS, Steffen, Eindhoven (NL)
(74) Representative: Philips Intellectual Property & Standards

(57) **Abstract**

Disclosed herein is a head coil device (100) comprising one or more magnetic resonance imaging coil elements and at least one light source (102) for a remote photoplethysmography. The at least one light source (102) is configured to illuminate at least one target area (106). The illuminating is configured for providing light (104) emitted by the at least one light source (102) and scattered at the target area (106) for a remote photoplethysmography signal (320) acquisition from the at least one target area (106).

## Description

### TECHNICAL FIELD

The invention relates to the field of magnetic resonance imaging, in particular it relates to a head coil device for magnetic resonance imaging.

### BACKGROUND

Photoplethysmography (PPG) is a technique that uses changes in light interaction, e.g., light absorption, to measure blood volume changes in the microvasculature. For example, a light source may be used to illuminate living tissue, such as the skin, mucous membranes, and other translucent tissues, and an optical sensor may be used to measure the amount of light that is scattered and/or absorbed by the tissue. However, when trying to use PPG in a magnetic resonance imaging system, problems may arise. These problems may, e.g., arise from space restrictions within a magnetic resonance imaging system and/or from strong magnetic and radio frequency fields generated within a bore of a magnetic resonance imaging system.

### SUMMARY

The invention provides for a head coil device, a medical system comprising a head coil device, a method for operating a medical system comprising a head coil device, and a computer program for performing the method for operating a medical system comprising a head coil device in the independent claims. Exemplary embodiments are given in the dependent claims.

In one aspect, a head coil device is disclosed. The head coil device comprises one or more magnetic resonance imaging coil elements and at least one light source for a remote photoplethysmography. The at least one light source is configured to illuminate at least one target area. The illuminating is configured for providing light emitted by the at least one light source and scattered at the target area for a remote photoplethysmography signal acquisition from the at least one target area.

In another aspect, a medical system is disclosed. The medical system comprises an example of the head coil device. The medical system further comprises a magnetic resonance imaging system configured for acquiring magnetic resonance data from a subject arranged at least partially with the head coil device within a bore of the magnetic resonance imaging system. The medical system further comprises at least one optical sensor configured to acquire optical sensor data comprising light emitted by the at least one light source of the head coil device. A field of view of the optical sensor covers at least part of an inner surface of the bore. The medical system further comprises a computational system. The computational system comprises a memory storing machine executable instructions for controlling the medical system.

Execution of the machine executable instructions causes the computational system to emit light by the at least one light source for illuminating the at least one target area. The execution further causes the computational system to receive the emitted light scattered at the at least one target area using the at least one optical sensor. The received light comprises emitted light, which is scattered additionally at the inner surface of the bore. The execution further causes the computational system to acquire a remote photoplethysmography signal using the received light.

In another aspect, a method of operating a medical system is disclosed. The medical system comprises an example of the head coil device. The medical system further comprises a magnetic resonance imaging system configured for acquiring magnetic resonance data from a subject arranged at least partially with the head coil device within a bore of the magnetic resonance imaging system. The medical system further comprises at least one optical sensor configured to acquire optical sensor data comprising light emitted by the at least one light source of the head coil device. The field of view of the optical sensor covers at least part of an inner surface of the bore. The medical system further comprises a computational system. The computational system comprises a memory storing machine executable instructions for controlling the medical system.

The method comprises emitting light by the at least one light source for illuminating the at least one target area. The method comprises receiving the emitted light scattered at the at least one target area using the at least one optical sensor. The received light comprises emitted light, which is scattered additionally at the inner surface of the bore. The method comprises acquiring a remote photoplethysmography signal using the received light.

In another aspect, a computer program is disclosed. The computer program comprises machine executable instructions for execution by a computational system. Execution of the machine executable instructions causes the computational system to perform an example of the method of operating a medical system.

### BRIEF DESCRIPTION OF THE DRAWINGS

In the following preferred embodiments of the invention will be described, by way of example only, and with reference to the drawings in which:
Fig. 1 shows an exemplary head coil device with an integrated light source;
Fig. 2 shows the exemplary head coil device with integrated light source of Fig. 1 from a different perspective;
Fig. 3 shows an exemplary MRI system with the exemplary head coil device of Fig. 1;
Fig. 4 shows an exemplary head coil device with an integrated light source;
Fig. 5 shows an exemplary head coil device with an integrated light source;
Fig. 6 shows an exemplary MRI system with exemplary head coil device with an integrated light source;
Fig. 7 shows an exemplary interior of an open exemplary head coil device with an integrated light source;
Fig. 8 shows an exemplary antiparallel circuit with two exemplary light emitting diodes;
Fig. 9 shows a flow chart illustrating an exemplary method for operating a medical system comprising an MRI system and a head coil device with an integrated light source;
Fig. 10 shows an exemplary computational system of an exemplary medical device; and
Fig. 11 shows an exemplary medical system comprising an MRI system, optical sensor, computational system, and head coil device with an integrated light source.

### DESCRIPTION OF EMBODIMENTS

Like numbered elements in these figures are either equivalent elements or perform the same function. Elements which have been discussed previously will not necessarily be discussed in later figures if the function is equivalent.

In an example, a head coil device is disclosed. The head coil device comprises one or more magnetic resonance imaging coil elements and at least one light source for a remote photoplethysmography. The at least one light source is configured to illuminate at least one target area. The illuminating is configured for providing light emitted by the at least one light source and scattered at the target area for a remote photoplethysmography signal acquisition from the at least one target area.

Examples may have the beneficial effect of providing a head coil device with an integrated light source. This light source may be configured for a remote photoplethysmography signal acquisition. The head coil device comprises one or more magnetic resonance imaging coil elements, which are configured for acquiring magnetic imaging data of a head and/or neck of a subject wearing the head coil device. For example, the magnetic resonance imaging coil elements are configured for acquiring magnetic imaging data of a brain and/or cervical spine of the subject.

The magnetic resonance imaging coil elements are configured as radiofrequency (RF) coils. They may be configured as transmitter and/or receiver RF coils. The RF coils may in particular be configured as transmitter and receiver coils, i.e., they may serve both transmit and receive functions.

When used as a transmitter, an RF coil in magnetic resonance imaging generates an oscillating and/or rotating magnetic field, referred to as B 1 field, that is oriented perpendicular to a static main magnetic field, referred to as B0 field. If the oscillation of the B1 field closely enough matches a natural precession of nuclear spins near the Larmor frequency, energy is deposited into the spin system, which causes a change in its net alignment. The B 1 field may typically be turned on for only brief periods of time, e.g., a few msec, referred to as RF pulses. By adjusting the magnitude and/or duration of these B1 pulses, the nuclear spin system may, e.g., be rotated by variable flip angles, such as 90° or 180°.

When used as a receiver, an RF coil is used for detecting a magnetic resonance imaging signal. An oscillating net magnetic flux from the nuclear spin system excited by the B 1 field may be detected by the receiver RF coil, in which an induced electric current is generated due to the oscillating net magnetic flux. This current may be amplified, digitized, and filtered to extract frequency and phase information of the oscillating net magnetic flux.

For example, the magnetic resonance imaging coil elements may comprise RF coils arranged in an array configured in parallel imaging applications. Parallel imaging uses knowledge about placement and sensitivities of receiver RF to assist spatial localization of the MR signal. For example, the RF coils may be used for parallel imaging applications like, e.g., sensitivity encoding (SENSE), which is based on the fact that receiver sensitivity may generally have an encoding effect complementary to Fourier preparation by linear field gradients. Thus, by using multiple receiver RF coils in parallel scan time in Fourier imaging may be reduced.

The light source integrated into the at least head coil device is configured for enabling a remote photoplethysmography. For example, one light source is integrated into the head coil device. For example, a plurality of light source, i.e., two or more light source, are integrated into the head coil device. The at least one light source is configured to illuminate at least one target area, i.e., a target area within a reception of the head coil device. The reception of the head coil device is configured to receive, at least partially, a subject's head and/or neck at a predefined position within the head coil device. The reception of the head coil device is, e.g., configured to receive the subject's head at a predefined position within the head coil device. The reception of the head coil device is, e.g., configured to receive the subject's neck at a predefined position within the head coil device.

The target area within the reception of the head coil device is selected such that it coincides with a specific skin area of a subject, which head and/or neck is, at least partially, arranged within the reception of the head coil device. When illuminating the target area, light is emitted by the at least one light source onto the target area, i.e., the specific skin area of the subject, and scattered at the target area comprising the specific skin area. This light scattered at the skin area is used for a remote photoplethysmography signal acquisition from the at least one target area, i.e., the respective skin area. The acquired remote photoplethysmography signal may, e.g., be used for monitoring the subject during a magnetic resonance imaging data acquisition, e.g., the subject's heart rate and/or respiratory rate. This remote photoplethysmography signal may, e.g., be used for triggering the magnetic resonance imaging data acquisition depending on, e.g., the subject's cardiac cycle and/or respiration.

Remote photoplethysmography (rPPG) refers to a remote, i.e., non-contact detection of pulsating blood propagating in the cardiovascular system which changes the blood volume in the microvascular tissue bed under the skin with each heartbeat, generating periodic changes of intensity and color of light scattered at the skin.

An optical sensor may be used to acquire optical sensor data comprising light scattered at a target area of a person's skin. The optical sensor data acquired over time may be used for providing a video stream of light scattered at the target area and extract subtle color changes in the skin to generate a remote PPG signal caused by changes in light absorption. Using rPPG, no physical sensor is required to be in contact with the skin. This may have the beneficial effect that using rPPG a PPG signal of a subject arranged within a magnetic resonance imaging system may be acquired without a physical sensor being arranged in contact with the subject, e.g., within a bore of the magnetic resonance imaging subject. Thereby, problems in view of space restrictions within the magnetic resonance imaging system and/or strong magnetic and radio frequency fields generated within the bore of the magnetic resonance imaging system may be avoided for the sensor.

With each cardiac cycle blood is pumped to the periphery by the heart resulting in a pulsed blood flow through the skin tissue. A plethysmogram optically obtained using rPPG may be used to detect blood volume changes in the microvascular bed of skin tissue resulting from this pulsed blood flow. Because blood flow to the skin may also be modulated by other physiological processes, like, e.g., respiration, the rPPG may further be used to monitor such other physical processes.

Since the skin is richly perfused, it is in general relatively easy to detect the pulsatile component of the cardiac cycle. A DC component of the signal may be attributable to the bulk absorption of the skin tissue, while an AC component may be attributable to variation in blood volume in the skin caused by the pressure pulse of the cardiac cycle. A height of the AC component of the photoplethysmogram may be proportional to the pulse pressure, i.e., the difference between the systolic and diastolic pressure in the arteries.

Respiration, e.g., affects the cardiac cycle by varying the intrapleural pressure, i.e., the pressure between the thoracic wall and the lungs. Since the heart resides in the thoracic cavity between the lungs, the partial pressure of inhaling and exhaling influences the pressure on the vena cava and the filling of the right atrium. During inspiration, the intrapleural pressure decreases, which distends the right atrium, allowing for faster filling from the vena cava, increasing ventricular preload, but decreasing stroke volume. Conversely during expiration, the heart is compressed, decreasing cardiac efficiency and increasing stroke volume.

Information about physiological processes, like cardiac cycle and/or respiration, determined using the rPPG signal may be used for triggering an MRI data acquisition depending on the respective physiological process.

For example, the target area is a target area within a head and neck region of a subject. For example, the at least one light source is configured to illuminate the target area, when the head of the subject is arranged at a predefined position within the head coil device. Light of the at least one light source, used as an rPPG light source, is scattered at the target area within the head and neck region of the subject, e.g., at the subject's forehead.

The subject's head and/or neck may be a beneficial region for acquiring an rPPG signal, while executing a magnetic resonance imaging data acquisition using a head coil device. When using a head coil device, the subject's head and neck are arranged at a position defined by the head coil device. Furthermore, the subject will be required to keep the head still. Furthermore, the head and neck region is in general at least partially exposed, when arranged within the head coil device. Head coil devices are in general designed such they comprise at least one opening, which exposes at least a section of the subject's face. The subject is enabled to see through that opening, reducing a risk of a claustrophobic sensation by the subject. Due to such openings, the subject's head and/or neck may be easily accessible for acquiring an rPPG signal, i.e., for applying light to this region as well as for acquiring light scattered within this region. In particular, the forehead may be advantageously used as a target area for rPPG, since the skin of the forehead is easily accessible and barely disturbed by facial motions, when the subject relaxes.

The scattered light may exit through one or more openings of the head coil device, such that it can be detected by an optical sensor. The scattered light exiting the head coil device may, e.g., further be scattered at an inner surface of a bore of magnetic resonance imaging system, when the head coil device is arranged within this bore. Thus, finally, e.g., an entire scene within the bore may be illuminated through multiple scattering of the light emitted by the at least one light source.

The scattered light carries an rPPG signal from the subject's skin within the target area and may be detected using the optical sensor. Due to the multiple scattering the scattered light carrying the rPPG signal is spread over a larger section of the field of view, e.g., over the entire field of view of the optical sensor. The section of the field of view over which the scattered light is spread due to the multiple scattering may in particular be larger than a section of the field of view over which the scattered light would be spread without the multiple scattering, i.e., the section of the field of view corresponding the target area. Thus, a large fraction of pixels of the optical sensor may be used for detected scattered light carrying the rPPG signal. By increasing the section of the field of view, i.e., the fraction of pixels of the optical sensor receiving scattered light from the target area the signal-to-noise-ratio (SNR) may be increased.

Examples may enable a reception of light scattered at the target zone, even in case the target zone is not part of the field of view of the optical sensor, i.e., in case there is no direct line of sight from the optical sensor to the target area. The scattered light may be redirected to the optical sensor, e.g., by the additional scattering at the inner surface of the bore of the magnetic resonance imaging system.

For the redirecting the scattered light to the optical sensor, e.g., no mirror may be required. The light scattered at the target area may rather be redirected by the additional scattering at the inner surface of the bore. Thus, the bore may be illuminated with the multiply scattered light. An optical sensor with a field of view corresponding to the bore and/or an opening of the bore of the magnetic resonance imaging may thus be able to receive a large portion of the light emitted by the integrated at least one light source.

Examples may allow for using an optical sensor for acquiring the rPPG signal, which is arranged outside a bore of a magnetic resonance system, e.g., at an opening of the bore. For example, the optical sensor may be arranged such that a field of view of the optical sensor covers the opening of the bore. For example, the optical sensor may be arranged such that the field of view of the optical sensor corresponds to the opening of the bore, i.e., may be mainly filled with the opening of the bore. Thus, examples may avoid typical problems arising from an integration of an optical sensor into a bore of a magnetic resonance imaging system. One of these problems is, e.g., due to space restrictions within a magnetic resonance imaging system, which collide with space requirements by the optical sensor. Further problems, which may be avoided, may, e.g., be caused by strong magnetic and RF fields generated within the bore of a magnetic resonance imaging system.

The optical sensor may in addition to the rPPG signal detection be used to monitor patient motion. Furthermore, the optical sensor may, e.g., be used for a presence detection of the subject including patient dimensions and patient pose, and/or of auxiliary devices.

For example, the rPPG may provide a live cardiac signal of the subject with at least a heart rate enabling, e.g., a monitoring of the heart rate. In particular, the heart rate may be acquired without a need for a usage of any ECG patches. Such a cardiac signal may, e.g., provide for a patient monitoring and may, e.g., allow to observe whether the patient becomes uncomfortable or nervous during a magnetic resonance data acquisition procedure.

Such a cardiac signal may, e.g., be used for a cardiac triggering a magnetic resonance imaging data acquisition using a magnetic resonance imaging system.

Using a cardiac triggering of a magnetic resonance imaging data acquisition may have the beneficial effect that, e.g., effects of a cardiac related pulsating of the brain and/or of a cardiac related pulsatile of cerebrospinal fluid CSF may be compensated. For example, cardiac related negative effects on a diffusion-weighted magnetic resonance imaging (DWI) may be compensated. DWI refers to a specific approach, in which magnetic resonance images are generated from data that uses a diffusion of water molecules to generate contrast in magnetic resonance images. DWI allows a mapping of a diffusion process of molecules, mainly water, in biological tissues, in vivo, and/or non-invasively. This molecular diffusion may reflect interactions with obstacles, such as, e.g., macromolecules, fibers, and membranes. Water molecule diffusion patterns may reveal microscopic details about tissue architecture, either normal or in a diseased state. A special kind of DWI is referred to as diffusion tensor imaging (DTI), which may be used to map white matter tractography in the brain.

For example, the rPPG may provide a live cardiac signal of the subject with at least a heart rate enabling, e.g., a monitoring of the heart rate. In particular, the heart rate may be acquired without a need for a usage of any ECG patches. Such a cardiac signal may, e.g., provide for patient monitoring and may, e.g., allow to observe whether the patient becomes uncomfortable or nervous during a magnetic resonance data acquisition procedure.

For example, the at least one light source is arranged at a rim of an opening of the head coil device. The opening is configured for exposing the at least one target area.

Examples may have the beneficial effect that the opening exposing the target area at the one hand side may facilitate the illumination of the target area using the at least one light source and at the other hand may facilitate an exiting of the head coil device by the light scattered at the target area.

For example, the head coil device is configured for receiving a head of the subject at a predefined position within the head coil device. Examples may have the beneficial effect that by receiving the head of the subject at the predefined position within the head coil device, the target area illuminated by the light source is fixed as well. The light source may be configured to illuminate a specific spot within the head coil device, which coincides with the predefined position, at which the head of the subject is received within the head coil device.

Thus, it may be ensured that the area illuminated by the at least one light source is the intended target area within a head and neck region of a subject.

For example, the opening is configured for exposing at least part of a face of the head received at the predefined position within the head coil device. The at least one target area is comprised by one of the following sections of the exposed part of the face: a forehead, a cheek.

For example, at least one target area is comprised by a forehead of the subject. The forehead may be a beneficial region for the target zone since it may be easily accessible due to a lack of coverage and may show the least motion of all facial skin. Furthermore, an opening exposing a subject's face may enable the subject to look out of the head coil devices, which may increase comfort and reduce a risk of a claustrophobic sensation by the subject.

For example, at least one target area is comprised by a cheek of the subject. The forehead may be a beneficial region for the target zone, since they provide comparable large section of facial skin, which may be usable for rPPG. Furthermore, an opening exposing a subject's face may enable the subject to look out of the head coil devices, which may increase comfort and reduce a risk of a claustrophobic sensation by the subject.

For example, the opening is configured for exposing at least part of a top region of the head received at the predefined position within the head coil device. The at least one target area is comprised by the exposed part of the top region of the head. The top region of the head of the subject comprising one or more of the following: a top frontal section of the head, a top mid-scalp section of the head, a top crown section of the head.

A target area comprised by the exposed part of the top region of the subject's head may in particular be beneficial in case of a subject without hair in this region. The skin in this region, i.e., the scalp, may be beneficial, since it shows hardly any motion as long as the head rests in its predefined position. Furthermore, an opening positioned such that it exposes at least part of a top region of the head within the head coil device may be in the line of sight of an optical sensor arranged at an end of a bore of a magnetic resonance imaging system. Thus, the opening may be part of the field of view of the optical sensor, facilitating a reception of light scattered at the target area within the respective opening.

Furthermore, a risk of blinding the subject by the light of the at least one light source may be low, when illuminating the top region of the subject's head.

For example, the opening is configured for exposing at least part of a neck of the subject with the head received at the predefined position within the head coil device. The at least one target area is comprised by the exposed part of the neck.

Examples may the beneficial effect that the skin at the neck is comparably unmoved. Furthermore, the risk of blinding the subject by the light of the at least one light source may be low, when illuminating the subject's neck.

For example, the at least one light source comprises at least one light emitting diode.

For example, the at least one light source comprises at least two light emitting diodes. Using two or more light emitting diodes may have the beneficial effect of providing different diodes, which may be controlled such that they are used during different phases of a magnetic resonance imaging data acquisition.

For example, the light emitting diodes are arranged in an antiparallel circuit integrated into a tuning circuit of the head coil device configured for tuning and detuning at least one coil element of the one or more of the coil elements.

Examples may have the beneficial effect that by integrating the light emitting diodes into the tuning circuit of the head coil device, the tuning circuit may be used for powering the light emitting diodes. Thus, no additional power supply and/or additional wires for supplying the light emitting diodes with power may be required. Using the tuning circuit for supplying the light emitting diodes may therefore avoid additional effort required to integrate any wires in an RF safe way and allow for a space saving integration of the diodes into the head coil device.

In magnetic resonance imaging, a main magnetic field is applied, which is referred to as the B0 field and acts to align the nuclei within a subject to be examined. By a relatively strong orthogonal transmit RF field, referred to as B 1 field, magnetic resonance is excited in the aligned nuclei. The B1 field causes the aligned nuclei or spins to tip into a plane orthogonal to the static magnetic field B0. Over time, the spins realign themselves with the B0 field emitting relatively weak RF resonance signals as they process. These RF resonance signals are detected by RF receiver coil elements tuned to the specific resonance frequency desired. The detected signals are passed to image processing equipment to reconstruct the detected signals into one or more magnetic resonance images.

The transmit RF signals, i.e., the transmit RF field, are in general orders of magnitude larger than the magnetic resonance signals generated by the excited nuclei within the subject and to be detected by receiver coil elements. To maintain patient safety and to protect the sensitive receiver equipment including the coil elements, the receiver coil elements are in general detuned during the transmit phase of a magnetic resonance imaging procedure. During receive phase of the magnetic resonance imaging procedure, the receiver coil elements are tuned to be able to detect the magnetic resonance signals generated by the excited nuclei.

Examples may allow for a powering of the light source, i.e., the light emitting diodes, with a minimal hardware change of the head coil device. The individual coil elements of the head coil device may be detuned and tuned during RF transmission and reception, respectively. Therefore, each coil element may, e.g., comprise a circuit including a PIN diode that is supplied with a detuning/tuning signal for detuning/tuning the respective coil element. The detuning signal may, e.g., be a positive voltage of, e.g., +5V, while the tuning signal may, e.g., be a negative voltage of, e.g., -12V. Thus, by switching the voltage supplied to a tuning circuit of a coil element, the respective coil element may be detuned and tuned during RF transmission and reception, respectively. The wires providing this voltage extend through the head coil device and may beneficially been used for supplying the light emitting diodes with power.

An antiparallel integration of the light emitting diodes refers to a connection of the diodes in parallel, but with their polarities reversed. Using an antiparallel integration of the light emitting diodes, e.g., of two light emitting diodes or two groups of light emitting diodes, may result in one of the light emitting diodes or one group of light emitting diodes being active during the supply of the detuning signal, i.e., a detuning voltage, while the another one of the light emitting diodes or another group of light emitting diodes is active during the supply of the tuning signal, i.e., a tuning voltage.

For example, the antiparallel circuit comprises one or more resistors configured for equalizing an intensity of the light emission by the light emitting diodes during a tuning operation mode and a detuning operation mode of the tuning circuit.

Using an antiparallel circuit one of the light emitting diodes or one group of the light emitting diodes may always, i.e., during detuning and during tuning phase, be switched on, while a magnetic resonance imaging data acquisition is executed. The resistors of the antiparallel circuit may be chosen such that the light output of the crossed light emitting diodes, i.e., the light emission, is equal during tuning and detuning phases of magnetic resonance imaging data acquisitions despite different supply voltages during the different operation modes of the tuning circuit, i.e., the during the tuning operation mode and the detuning operation mode.

In another example, a DC power supply for a pre-amplifier and digitization hardware, i.e., an RXE unit, of the head coil device configured for processing a received magnetic resonance imaging signal may be used to power the at least one light source.

For example, the at least one light source is configured to emit green light. Examples may have the beneficial effect that green light has a higher absorptivity of hemoglobin compared to other light. Due to the higher absorptivity, optical sensors may gauge the flow of blood and heart beats more accurately, when using green light. Thus, using green light, e.g., emitted by a green light emitting diode, may increase or even maximize the detectable rPPG signal. For example, the optical sensor may be configured for receiving green light.

For example, the at least one light source is configured to emit infrared light, e.g., near-infrared light. Using infrared light, e.g., near-infrared light, may have the beneficial effect, that a tissue penetration depth of infrared light is higher compared to visible light. Infrared light can, e.g., reach dermal, subcutaneous, and deeper layers. Furthermore, by using light outside the visible spectrum a risk of blinding the subject by the light of the at least one light source may be avoided. For example, the optical sensor may be configured for receiving infrared light.

For example, the at least one light source is configured to emit white light. For example, the optical sensor may be configured for receiving white light.

For example, the head coil device comprises no additional mirror for reflecting at least part of the light emitted by the at least one light source and scattered at the target area. Instead of using a mirror for redirecting the scattered light, an additional scattering of the light at an inner surface of a bore of a magnetic resonance imaging system, in which the head coil device is arranged, may be used.

Thus, any additional effort in development and manufacturing caused by a mirror as well as any additions to the clinical workflow may be avoided. Such additions, which may be avoided comprise a precise relative positioning of patient, mirror, and optical sensor. Furthermore, any risks of a collision of the mirror with a bore of a magnetic resonance imaging system, when the head coil device with the mirror is arranged within the respective bore, may be avoided.

For example, a medical system is disclosed. The medical system comprises an example of the head coil device as described above. The medical system further comprises a magnetic resonance imaging system configured for acquiring magnetic resonance data from a subject arranged at least partially with the head coil device within a bore of the magnetic resonance imaging system. The medical system further comprises at least one optical sensor configured to acquire optical sensor data comprising light emitted by the at least one light source of the head coil device. A field of view of the optical sensor covers at least part of an inner surface of the bore. The medical system further comprises a computational system. The computational system comprises a memory storing machine executable instructions for controlling the medical system.

Execution of the machine executable instructions causes the computational system to emit light by the at least one light source for illuminating the at least one target area. The execution further causes the computational system to receive the emitted light scattered at the at least one target area using the at least one optical sensor. The received light comprises emitted light, which is scattered additionally at the inner surface of the bore. The execution further causes the computational system to acquire a remote photoplethysmography signal using the received light.

Examples may enable an acquisition of an rPPG signal with a good signal-to-noise ratio.

For example, the target area is a target area within a head and neck region of a subject. For example, the at least one light source is configured to illuminate the target area, when the head of the subject is arranged at a predefined position within the head coil device.

For example, the medical system comprises no additional mirror for reflecting at least part of the light emitted by the at least one light source.

For example, the execution of the machine executable instructions further causes the computational system to use the acquired remote photoplethysmography signal for a cardiac triggering of a magnetic resonance imaging data acquisition executed using the magnetic resonance imaging system.

Magnetic resonance imaging head scans may benefit from a cardiac triggering. Such a cardiac triggering may be enabled using the rPPG signal.

For example, a method of operating a medical system is disclosed. The medical system comprises an example of the head coil device. The medical system further comprises a magnetic resonance imaging system configured for acquiring magnetic resonance data from a subject arranged at least partially with the head coil device within a bore of the magnetic resonance imaging system. The medical system further comprises at least one optical sensor configured to acquire optical sensor data comprising light emitted by the at least one light source of the head coil device. A field of view of the optical sensor covers at least part of an inner surface of the bore. The medical system further comprises a computational system. The computational system comprises a memory storing machine executable instructions for controlling the medical system.

The method comprises emitting light by the at least one light source for illuminating the at least one target area. The method comprises receiving the emitted light scattered at the at least one target area using the at least one optical sensor. The received light comprises emitted light, which is scattered additionally at the inner surface of the bore. The method comprises acquiring a remote photoplethysmography signal using the received light.

Examples may enable an acquisition of an rPPG signal with a good signal-to-noise ratio.

For example, the target area is a target area within a head and neck region of a subject. For example, the at least one light source is configured to illuminate the target area, when the head of the subject is arranged at a predefined position within the head coil device.

The method may, e.g., be configured for operating any of the examples of a medical system described herein.

For example, the method further comprises using the acquired remote photoplethysmography signal for a cardiac triggering of a magnetic resonance imaging data acquisition executed using the magnetic resonance imaging system.

Magnetic resonance imaging head scans may benefit from a cardiac triggering. Such a cardiac triggering may be enabled using the rPPG signal.

In another aspect, a computer program comprises machine executable instructions for execution by a computational system. Execution of the machine executable instructions causes the computational system to perform an example of the method of operating a medical system.

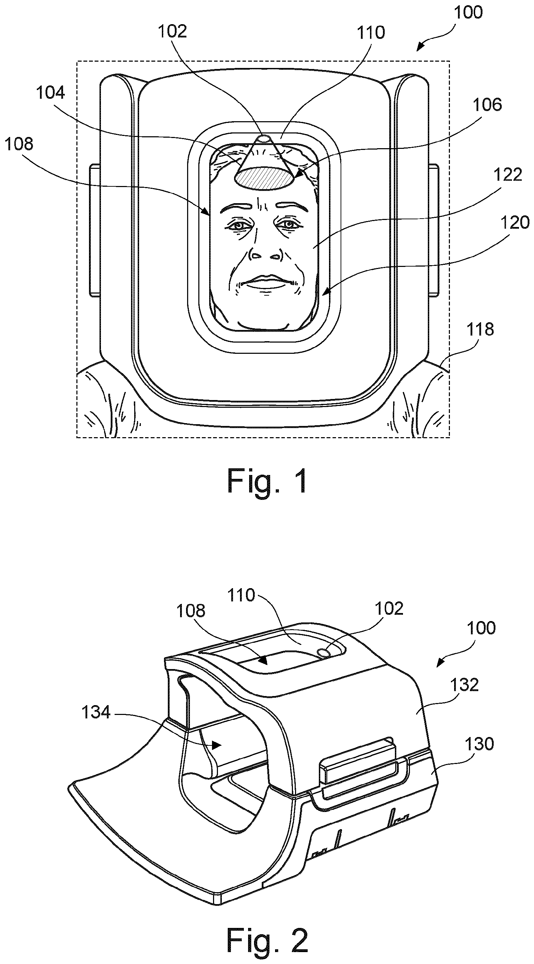

Fig. 1 shows an exemplary head coil device 100 with an integrated light source 102 for a remote photoplethysmography. The head coil device 100 comprises one or more magnetic resonance imaging coil elements and the at least one light source 102. The head coil device 100 is configured for receiving a head 122 of the subject 118 at a predefined position within the head coil device 100. For this purpose, the head coil device 100 comprises a reception, in which the head 122 of the subject 118 is received.

The light source 102 is configured to illuminate a target area 106. In the example shown in Fig. 1, the target area 106 is a target area within a head and neck region 120 of the subject 118, e.g.., the subject's forehead. The light source 102 is configured to illuminate this target area 106, when the head 122 of the subject 118 is arranged at the predefined position within the head coil device 100. The light source 102 emits light 104, which is scattered at the target area 106 for a remote photoplethysmography signal acquisition from the target area 106, i.e., the subject's 118 forehead. Thus, the light source 102 may be used as an rPPG light source.

The light source 102 may, e.g., be configured to emit one or more of the following: green light, infrared light, white light.

The exemplary head coil device of Fig. 1 is shown to comprise one light source 102. The head coil device 100 may, e.g., comprise more than one light source 102. In case of a plurality of light sources 102, the light sources 102 may be configured to illuminate the same target area 106 or different target areas 106. The light sources 102 may be arranged adjacent to each other or may be arranged at different positions spaced apart from each other.

The head coil device 100 comprises an opening 108. In the example of Fig. 1, the opening 108 exposes the subject's 118 face, when the subject's 118 head 122 is arranged at the predefined position within the head coil device 100. The opening 108 enables the subject 118 to see through the respective opening 108 out of the head coil device 100. This may increase the subject's 118 comfort during the magnetic resonance imaging data acquisition. It may in particular reduce a risk of a claustrophobic sensation by the subject 118. Due to such openings 108, the subject's head and/or neck may be easily accessible for acquiring an rPPG signal of the subject 118.

The light source 102 is arranged at a rim 110 of the opening 108, e.g., at a top rim of the opening 108, which exposes at least part of a face of the subject's 118 head 122 received at the predefined position within the head coil device 100. In the example of Fig. 1, the target area 106 is the forehead of the subject 118. The light source 102 may, e.g., be configured to illuminate only the forehead and not the eyes of the subject. Thus, it may be avoided that the subject is blinded and/or safety hazards may be avoided. Additionally or alternatively, the target area 106 could also be comprised by other exposed parts of the face, e.g., the subject's 118 cheeks.

The light 104 emitted by the integrated light source 102 is scattered at the target area 106 and may be received by an optical sensor (not shown) for an rPPG signal acquisition. This rPPG light source 102 is integrated into the head coil device 100. The light source 102 may, e.g., comprise one or more light emitting diodes. The opening 108 may not only allow for illuminating the target area 106 by the light source 102, but further for the emitted light 104, which is scattered at the target area 106, to exit the head coil device 100.

Fig. 2 shows an exemplary head coil device 100 with integrated light source 102 of Fig. 1 from a lateral perspective. As can be seen, the head coil device comprises two parts, i.e., a lower part 130 and an upper part 132. These two parts 130, 132 form a reception 134, which is configured to receive subject's head at a predefined position within. The upper part 132 may, e.g., comprise the light source 102. The upper part 132 may, e.g., be detachable from the lower part 130 for arranging the subject's head onto the lower part 130 in the predefined position. Then, the upper part 132 may be assembled on the lower part 130, such that the light source 102 of the upper part 132 is aligned with target area 106, when the subject's head is arranged at the predefined position.

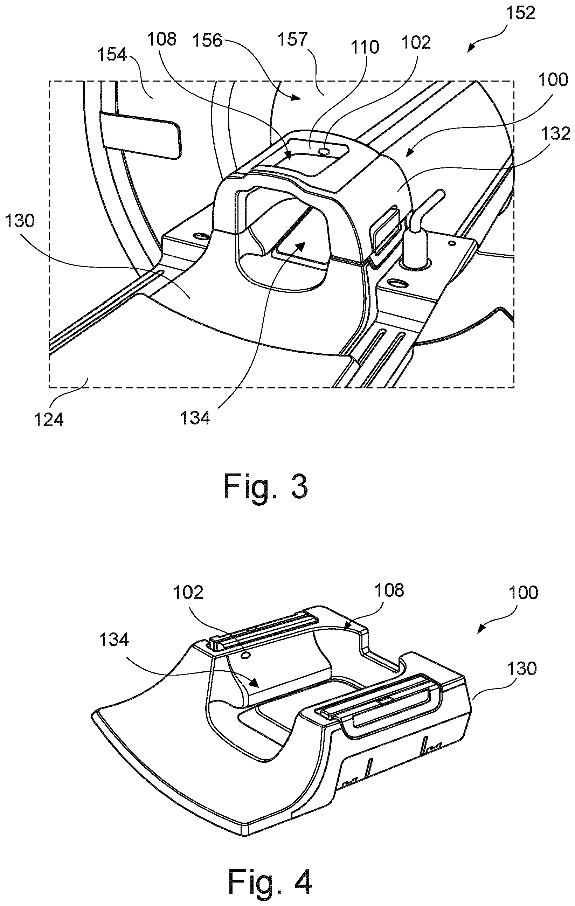

Fig. 3 shows an exemplary magnetic resonance imaging system 152 with the exemplary head coil device 100 of Fig. 1. The head coil device 100 is mounted with its lower part 132 onto a subject support 124, which is configured as an examination table. A subject may lay down onto the subject support 124 such that the subject's head rests on the lower part 130 of the head coil device 100 in a predefined position. The subject support 124 with the subject may be moved into a bore 156 of a main magnet 154 of the magnetic resonance imaging system 152 for acquiring magnetic resonance imaging data of the subject. When the head coil device 100 is arranged within the bore 156, light emitted by the light source 102 integrated into the head coil device 100, e.g., into the upper part 132, may be scattered at the subject's head within the target area. The scattered light may exit the head coil device 100 through the opening 108 and be additionally scattered at an inner surface 157 of the bore 156. Thus, the interior of the bore 156 may be illuminated with the scattered light emitted by the light source 102. This multiply scattered light may be received by an optical sensor (not shown), which covers the bore 156 and/or an opening of the bore 156 with its field of view. Due to the scattering, the light may be spread and cover a larger portion of the field of view of the optical sensor than the target area itself would. Thus, the scattered light may be received by a larger number pixels of the optical sensor than a number of pixels corresponding to the size of the target area. Thus, a signal-to-noise ratio may be increased compared to a scenario without scattering. Furthermore, due to the scattering, light from the target area may even be received by the optical sensor in case the target area is not in line of sight of the optical sensor, i.e., in case the target area itself cannot be seen by the optical sensor.

Fig. 4 shows another exemplary head coil device 100 with an integrated light source 102. The form of the head coil device 100 of Fig. 4, e.g., corresponds to the form of the lower part 130 of the head coil device 100 of Fig. 1. The head coil device 100 is configured to receive a subject's head in a predefined position within the reception 134. In a lateral sidewall of the head coil device 100, the integrated light source 102 is integrated. The light source 102 is, e.g., arranged and configured to illuminate a target area, which comprises the subject's cheek. The reception 134 is open upwards forming an opening 108. Thus, light emitted by the light source 102 and scattered at the subject's cheek as arranged within the target area may exit the head coil device 100 via the opening 108.

Alternatively, the light source 102 may, e.g., be arranged and configured to illuminate a target area, which comprises the subject's neck. The opening 108 may expose at least part of a neck of the subject arranged at the predefined position within the head coil device 100, i.e., the light emitted by the light source 102 and scattered at the subject's neck as arranged within the target area may exit the head coil device 100 via the opening 108.

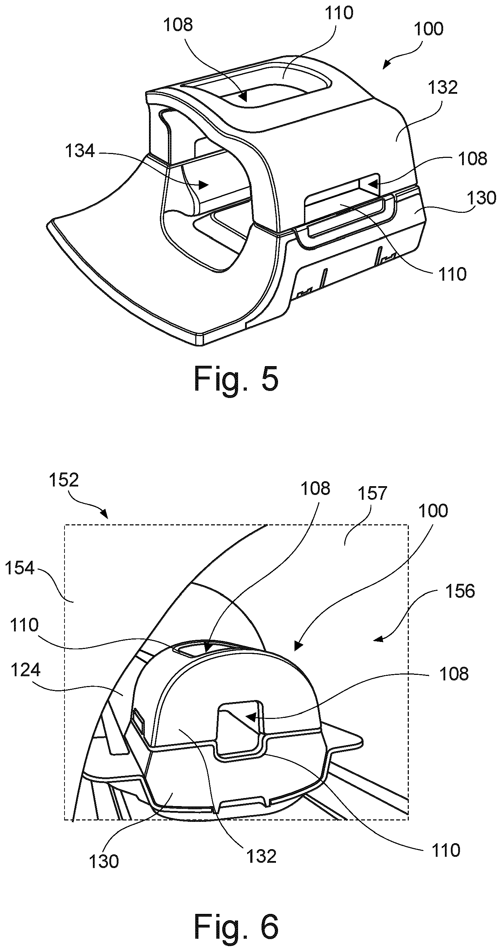

Fig. 5 shows another exemplary head coil device 100 with an integrated light source 102. The form of the head coil device 100 of Fig. 5, e.g., corresponds to the form of the head coil device 100 of Fig. 1 with the only exception, that the head coil device 100 of Fig. 5 comprises an additional lateral opening 108.

The lateral opening 108 of the coil device 100 may, e.g., expose at least part of a neck of the subject arranged at the predefined position within the head coil device 100. The integrated light source 102 may be arranged at a rim 110 of the lateral opening 108. The light source 102 may, e.g., be arranged and configured to illuminate a target area, which comprises the subject's neck. Light emitted by the light source 102 and scattered at the subject's neck as arranged within the target area may exit the head coil device 100 via the lateral opening 108. The light exiting the head coil device 100 via the lateral opening 108 may, e.g., be additionally scattered at an inner surface of a bore of a magnetic resonance imaging system and received by an optical sensor (not shown) with a field of view covering the bore and/or an opening of the bore.

The lateral opening 108 of the coil device 100 may, e.g., expose at least part of a check of the subject arranged at the predefined position within the head coil device 100. The integrated light source 102 arranged at the rim 110 of the lateral opening 108 may, e.g., be arranged and configured to illuminate a target area, which comprises the subject's cheek. Light emitted by the light source 102 and scattered at the subject's cheek as arranged within the target area may exit the head coil device 100 via the lateral opening 108. Furthermore, light scattered at the subject's cheek may also exit the head coil device 100 via the upper opening 108, which exposes the subject's face. The light exiting the head coil device 100 via the lateral opening 108 may, e.g., be additionally scattered at an inner surface of a bore of a magnetic resonance imaging system and received by an optical sensor (not shown) with a field of view covering the bore and/or an opening of the bore.

Fig. 6 shows an exemplary magnetic resonance imaging system 152 with an exemplary head coil device 100, which comprises an integrated light source 102. The magnetic resonance imaging system 152 and an exemplary head coil device 100 of Fig. 6 may, e.g., be identical with the magnetic resonance imaging system 152 and an exemplary head coil device 100 of Fig. 3. In this case, Fig. 6 shows a view into the bore 156 of the magnetic resonance imaging system 152 from an opposite side compared to Fig. 3. For example, an optical sensor may be arranged at this opposite end of the bore 156 and the view illustrated in Fig. 6 may correspond to the field of view of the optical sensor. The optical sensor may be configured to receive light scattered at a target area within the head coil device 100 and further scattered at the inner surface 157 of the bore 156.

Alternatively or additional, the light source 102 or an additional light source, respectively, may, e.g., be arranged at a rim 110 of a top opening 108 of the head coil device 100. This top opening 108 faces the bore 156 and the direction into the bore 156. The top opening 108 may expose at least part of a top region of a head of the subject arranged at a predefined position within the head coil device 100. The at least one target area, which is illuminated by light emitted by the integrated light source arranged at the rim 100 of the top opening 108, may be comprised by the exposed part of the top region of the head. This top region of the head of the subject may, e.g., comprising one or more of the following: a top frontal section of the head, a top mid-scalp section of the head, a top crown section of the head.

The target area may, in this case, e.g., be directly visible for an optical sensor through the top opening 108. For example, an optical sensor may be arranged at this opposite end of the bore 156 and the view illustrated in Fig. 6 may correspond to the field of view of the optical sensor. Thus, the optical sensor may be configured to look directly onto the target area via the top opening 108. The optical sensor may be configured to receive light scattered at a target area within the head coil device 100 and exiting the head coil device 100 via the top opening 108. Additionally, at least some of the light exiting the head coil device 100 via the top opening 108 and being received by the optical sensor may, e.g., further be scattered at the inner surface 157 of the bore 156.

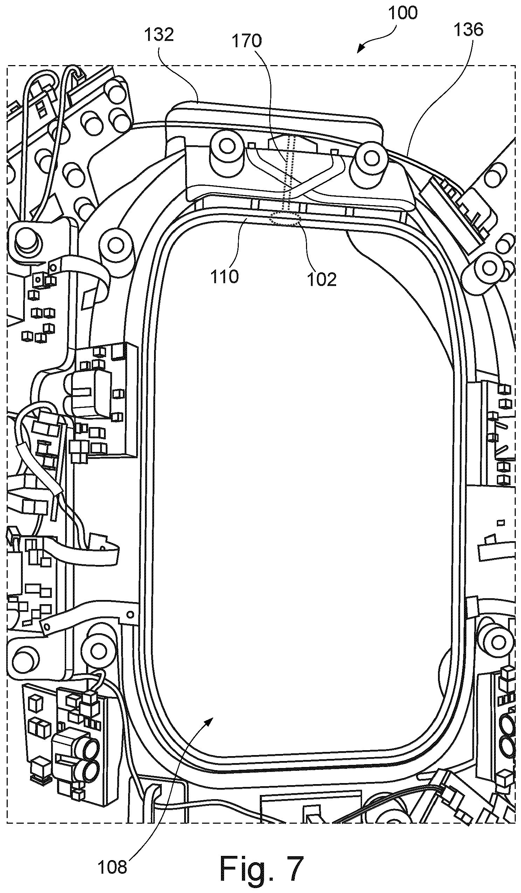

Fig. 7 shows an exemplary interior of an open exemplary head coil device 100 with an integrated light source 102. The head coil device 100 is shown without its top cover. The interior may, e.g., be an interior of an upper part 132 of a head coil device 100, e.g., a head coil device 100 as shown in Fig. 1. The head coil device 100 comprises a tuning circuit 136 configured for tuning and detuning at least one coil element of the one or more of the coil elements of the head coil device 100. This tuning circuit 136 comprises one or more wires arranged in the vicinity of a rim 110 of an opening 108 of the head coil device 100. This wiring 136 providing a detuning/tuning signal for the coil elements of the head coil device 100 may, e.g., be routed exactly via a location required for the light source 102. Thus, the light source 102 may be arranged at the rim 110 of the opening 108 and conductively connected with the tuning circuit 136 via an illumination circuit 170. This illumination circuit 170 may, e.g., be of a few mm size, such that for integrating the light source 102 into the head coil device 100 a hardware modification may be kept minimal.

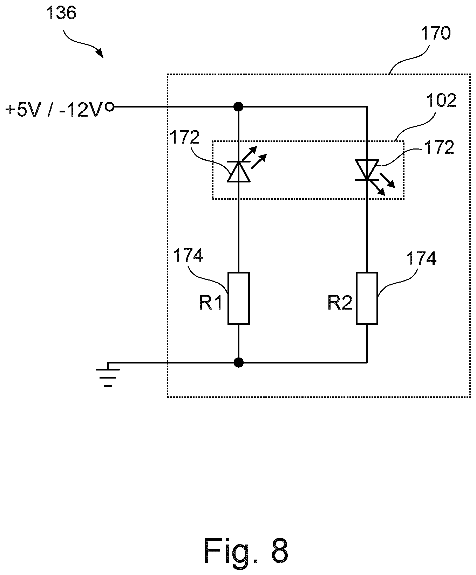

Fig. 8 shows an exemplary antiparallel illumination circuit 170 with a light source 102 comprising two exemplary light emitting diodes 172. The light emitting diodes 172 are arranged in the antiparallel circuit 170, with may be integrated into a tuning circuit 136 of the head coil device. This tuning circuit 136 is configured for tuning and detuning at least one coil element of the one or more of the coil elements of the head coil device. The antiparallel circuit 170 further comprises two resistors 174, which are configured for equalizing an intensity of the light emission by the two light emitting diodes 172 during a tuning operation mode and a detuning operation mode of the tuning circuit 136. Thus, during the tuning operation mode one of the two light emitting diodes 172 may be activated and emitting light, while during the detuning operation mode the other one of the two light emitting diodes 172 may be activated and emitting light. Even in case of different voltages being applied to the tuning circuit 136 during the tuning operation mode and the detuning operation mode of the tuning circuit 136, the light emission by the active light emitting diodes 172 may be kept constant, when the light emitting diodes 172 are switched due to a switching of the operation mode of the tuning circuit 136. By keeping the light emission constant an influence of the switching of the operation mode of the tuning circuit 136 on the rPPG signal may be prevented or kept minimal.

The individual coil elements of the head coil device 100 may be detuned and tuned during RF transmission and reception, respectively. Therefore, each coil element may, e.g., contain a circuit including, e.g., a PIN diode, that is supplied with a detuning/tuning signal. The detuning signal may, e.g., be a voltage of +5V, while a tuning signal may, e.g., be a voltage of -12V. It is proposed to integrate the light source 102, e.g., in the form of two crossed, i.e., antiparallel connected light emitting diodes 172 in combination with suitable resistors 174. Theses resistors 174 may be configured as current limiters. Thus, in the configuration shown in Fig. 8 at least one of these light emitting diodes 172 may always be switched on as long as the tuning circuit 136 is operated in one of the two modes. The resistors 174 may be chosen such that the light emission of the two crossed light emitting diodes 172 is equal despite their different supply voltage in the different operation modes of the tuning circuit 136.

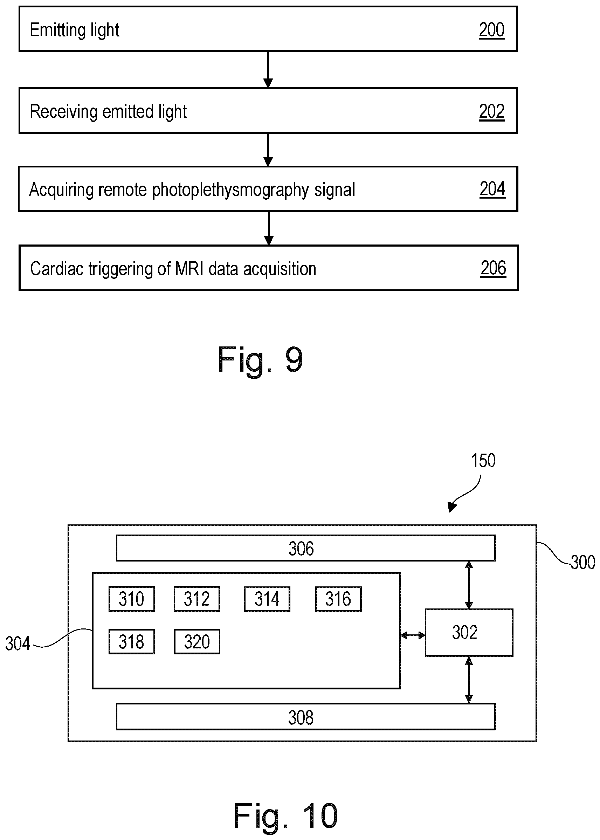

Fig. 9 shows a flow chart illustrating an exemplary method for operating a medical system comprising a magnetic resonance imaging system and a head coil device with an integrated light source. The head coil device may comprise one or more magnetic resonance imaging coil elements and at least one light source for a remote photoplethysmography. The at least one light source is configured to illuminate at least one target area. The illuminating is configured for providing light emitted by the at least one light source and scattered at the target area for a remote photoplethysmography signal acquisition from the at least one target area.

For example, the target area is a target area within a head and neck region of a subject. For example, the at least one light source is configured to illuminate the target area, when the head of the subject is arranged at a predefined position within the head coil device.

The magnetic resonance imaging system comprised by the medical system is configured for acquiring magnetic resonance data from a subject arranged at least partially with the head coil device within a bore of the magnetic resonance imaging system. The medical system further comprises at least one optical sensor configured to acquire optical sensor data comprising light emitted by the at least one light source of the head coil device. A field of view of the optical sensor covers at least part of an inner surface of the bore. The medical system further comprises a computational system. The computational system comprises a memory storing machine executable instructions for controlling the medical system.

In block 200, light is emitted by the at least one light source for illuminating the at least one target area. In block 202, the emitted light scattered at the at least one target area is received using the at least one optical sensor. The received light comprises emitted light, which is scattered additionally at the inner surface of the bore. In block 204, a remote photoplethysmography signal is acquired using the received light. In block 206, the remote photoplethysmography signal acquired in block 204 is used for a cardiac triggering of a magnetic resonance imaging data acquisition executed using the magnetic resonance imaging system.

Fig. 10 shows an exemplary computational system 300 of an exemplary medical device 150. The computational system 300 may, e.g., be configured for acquiring an rPPG signal 320 and/or for using the acquired rPPG signal 320 for a cardiac triggering of a magnetic resonance imaging data acquisition executed using a magnetic resonance imaging system.

The computational system 300 is shown as comprising a computational component 302. The computational component 302 is intended to represent one or more processors or processing cores or other computational elements. The computational component 302 is shown as being connected to a hardware interface 306 and a memory 304. The hardware interface 306 may enable the computational component 302 to exchange commands and data with other components, e.g., a head coil device, an optical sensor and/or a magnetic resonance imaging system. The hardware interface 306 may, e.g., enable the computational component 302 to control the optical sensor to acquire optical sensor data 319, e.g., a sequence of optical images. The hardware interface 306 may, e.g., enable the computational component 302 to control a head coil device. The hardware interface 306 may in particular enable the computational component 302 to control an illumination of at least one target area within a head and neck region of a subject arranged at least partially at a predefined position within the head coil device. This illumination may comprise an emission of light by at least one light source integrated into the head coil device, which may be controlled by the computational component 302. The hardware interface 306 may, e.g., further enable the computational component 302 to control a magnetic resonance imaging system.

The computational system 300 is further shown as being connected to a user interface 308 which may for example enable an operator to control and operate the computational system 300 and via the computational system 300 a head coil device, an optical sensor and/or a magnetic resonance imaging system. The user interface 308 may, e.g., comprise an output and/or input device enabling the operator to interact with the computational system 300. The output device may, e.g., comprise a display device configured for displaying images of the optical sensor data 318, an rPPG signal 320 acquired using the optical sensor data 318 and/or of magnetic resonance images 316 reconstructed using magnetic resonance imaging data 314 acquired using a magnetic resonance imaging system. The input device may, e.g., comprise a keyboard and/or a mouse enabling the operator to insert control commands for controlling the computational system 300 and via the computational system 300 a head coil device, an optical sensor and/or a magnetic resonance imaging system.

The memory 304 is shown as containing machine-executable instructions 310. The machine-executable instructions 310 enable the computational component 302 to perform controlling tasks, such as controlling a head coil device, an optical sensor and/or a magnetic resonance imaging system, to perform numerical tasks, as well as performing various signal data processing tasks, acquiring the rPPG signal 320 and/or reconstructing the magnetic resonance images 316. The machine-executable instructions 310 may, e.g., enable the computational component 302 and thus the computational system 300 to execute the method for operating a medical system of Fig. 9.

The memory 304 is further shown as containing optical sensor data 318 received, e.g., from an optical sensor. The optical sensor data 318 may, e.g., comprise a sequence of optical images of light scattered at a target area of a subject. The memory 304 is further shown as containing an rPPG signal 320 acquired using the optical sensor data 318, i.e., extracted from the optical sensor data 318.

This rPPG signal 320 may be used for a cardiac triggering of a magnetic resonance imaging data acquisition executed using a magnetic resonance imaging system. For this purpose, the memory 304 may further contain one or more sets of pulse sequences 312 configured for controlling the magnetic resonance imaging data acquisition, which may be triggered using the rPPG signal 320. The memory 304 is further shown as containing magnetic resonance imaging data 314 acquired using the magnetic resonance imaging system, which is controlled using the one or more sets of pulse sequences 312. Finally, the memory 304 is shown as containing magnetic resonance images 316 reconstructed using the acquired magnetic resonance imaging data 314.

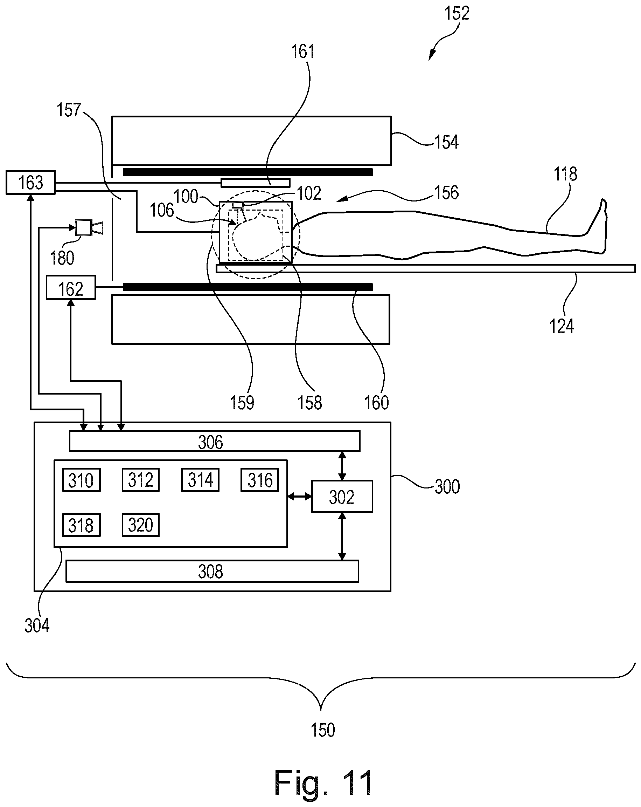

Fig. 11 shows an exemplary medical system 150 comprising a magnetic resonance imaging system 152, an optical sensor 180, a computational system 300, and a head coil device 100 with at least one integrated light source 102. The medical system 150 may, e.g., be operated according to the method of Fig. 9. The computational system 300 of Fig. 11 may, e.g., be the computational system 300 of Fig. 10.

The head coil device 100 may comprise one or more magnetic resonance imaging coil elements and at least one light source 102 for a remote photoplethysmography. The at least one light source 192 is configured to illuminate at least one target area 106. The illuminating is configured for providing light emitted by the at least one light source 102 and scattered at the target area 106 for an rPPG signal 320 acquisition from the at least one target area 106.

For example, the target area 106 is a target area within a head and neck region of a subject 118. For example, the at least one light source 102 is configured to illuminate the target area 106, when a head of the subject 118 is arranged at a predefined position within the head coil device 100. The target area 106 may, e.g., comprise one or more of the following tissue sections of the subject 118: a forehead, a check, a neck, and/or a top region of the head of the subject 118. The top region of the head of the subject 118 may, e.g., comprise one or more of the following: a top frontal section of the head, a top mid-scalp section of the head, a top crown section of the head.

The magnetic resonance imaging system 152 comprised by the medical system 150 is configured for acquiring magnetic resonance data from the subject 118 arranged at least partially with the head coil device 100 within a bore 156 of the magnetic resonance imaging system 152. The medical system 150 further comprises the at least one optical sensor 180 configured to acquire optical sensor data 318 comprising light emitted by the at least one light source 102 of the head coil device 100. A field of view of the optical sensor 180 covers at least part of an inner surface 157 of the bore 156. The light emitted by the at least one light source 102 and received by the optical sensor 180 is scattered at the target area 106 and may, e.g., be additionally scattered at the inner surface 157 of the bore 156.

The magnetic resonance imaging system 152 is being controlled by the hardware interface 306 of the computational system 300. The magnetic resonance imaging system 152 comprises a magnet 154. The magnet 154 is a superconducting cylindrical type of magnet with the bore 156 through it. The use of different types of magnets is also possible. For instance, it is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular because the subject is less confined. Inside the cryostat of the cylindrical magnet there is a collection of superconducting coils.

Within the bore 156 of the cylindrical magnet 154 there is an imaging zone 159, where the magnetic field is strong and uniform enough to perform magnetic resonance imaging. A field of view 158 is shown within the imaging zone 159. The magnetic resonance data is typically acquired for the field of view 158. A subject 118 is shown as being supported by a subject support 124, like an examination table, such that at least a portion of the subject 118, e.g., the head, is within the imaging zone 159 and the field of view 158. The head of the subject 118 is arranged within the head coil device 100.

Within the bore 156 of the magnet 154 there is also a set of magnetic field gradient coils 160 which is used for the acquisition of preliminary magnetic resonance imaging data to spatially encode magnetic spins within the bore 156 of the magnet 154. The magnetic field gradient coils 160 are connected to a gradient controller, i.e., a magnetic field gradient coil power supply 162. The magnetic field gradient coils 160 are intended to be representative. Typically, magnetic field gradient coils 160 contain three separate sets of coils for spatial encoding in three orthogonal spatial directions. The magnetic field gradient power supply 162 supplies current to the magnetic field gradient coils 160. The current supplied to the magnetic field gradient coils 160 is controlled as a function of time and may be ramped or pulsed.

Adjacent to the imaging zone 159 is a radio-frequency antenna 161 arranged for manipulating the orientations of magnetic spins within the imaging zone 159 and for receiving radio transmissions from spins also within the imaging zone 159. The radio-frequency antenna 161may contain multiple coil elements. The radio-frequency antenna 161 is connected to a radio-frequency transceiver 163. The radio-frequency antenna 161 and radio- frequency transceiver 163 may be replaced by separate transmit and receive coils and a separate transmitter and receiver. It is understood that the radio-frequency antenna 161 and the radio- frequency transceiver 163 are representative. The radio-frequency antenna 161 is intended to also represent a dedicated transmit antenna and a dedicated receive antenna. Likewise, the transceiver 163 may also represent a separate transmitter and receivers. The radio-frequency antenna 161 may also have multiple receive/transmit elements and the radio- frequency transceiver 163 may have multiple receive/transmit channels. The transceiver 163 and the gradient controller 162 are shown as being connected to the hardware interface 306 of the computational system 300.

The head coil device 100 with its coil elements may, e.g., be used in addition to the radio-frequency antenna 161 or may replace the radio-frequency antenna 161 at least partly. The head coil device 100 may, e.g., replace transmit and/or receive coils of the radio-frequency antenna 161.

The execution of the machine-executable instructions 310 by the computational system 300 may further cause the computational system 300 to acquire magnetic resonance imaging data 314 using the pulse sequences 312. The acquisition of the magnetic resonance imaging data 314 may be triggered using the rPPG signal 320. The acquired magnetic resonance imaging data 314 is used to reconstruct the magnetic resonance images 316. Additionally or alternatively, the rPPG signal 320 may be used to sort, collate, select and/or annotate the reconstructed magnetic resonance images 316, e.g., to show extracted PPG information or information derived therefrom, such as a cardiac phase, alongside the reconstructed magnetic resonance images 316.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer-readable medium(s) having computer executable code embodied thereon.

Any combination of one or more computer-readable medium(s) may be utilized. The computer-readable medium may be a computer-readable signal medium or a computer-readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer-readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid-state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer-readable-storage medium also refers to various types of recording media capable of being accessed by the computer device via a network or communication link. For example, data may be retrieved over a modem, over the internet, or over a local area network. Computer executable code embodied on a computer-readable medium may be transmitted using any appropriate medium, including but not limited to wireless, wire line, optical fiber cable, RF, etc., or any suitable combination of the foregoing.

A computer-readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer-readable signal medium may be any computer-readable medium that is not a computer-readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine-executable instruction or computer executable code. References to the computational system comprising the example of a `computational system' should be interpreted as possibly containing more than one computational component or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine-executable code or instructions may be executed by multiple computational systems or processors that may be within the same computing device or which may even be distributed across multiple computing devices.

Machine-executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++, or the like and conventional procedural programming languages, such as the "C" programming language or similar programming languages and compiled into machine-executable instructions. In some instances, the computer executable code may be in the form of a high-level language or in a pre-compiled form and be used in conjunction with an interpreter which generates the machine-executable instructions on the fly. In other instances, the machine-executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a Local Area Network (LAN) or a Wide Area Network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine-executable instructions or computer program instructions may also be stored in a computer-readable medium that can direct a computer, other programmable data processing apparatus, or other devices to function in a particular manner, such that the instructions stored in the computer-readable medium produce an article of manufacture including instructions which implement the function/act specified in the flowchart and/or block diagram block or blocks.

The machine-executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode Ray Tube (CRT), storage tube, bi-stable display, electronic paper, vector display, flat panel display, Vacuum Fluorescent (VF) display, Light-Emitting Diode (LED) displays, Electroluminescent display (ELD), Plasma Display Panels (PDP), Liquid Crystal Display (LCD), Organic Light-Emitting Diode (OLED) displays, a projector, and head-mounted display.

Magnetic resonance imaging data is defined herein as being the recorded measurements of RF signals emitted by atomic spins using the antenna of a magnetic resonance apparatus, like a magnetic resonance imaging system and/or a head coil device, during a magnetic resonance imaging data. A magnetic resonance image is defined herein as being the reconstructed two- or three-dimensional visualization of anatomic data contained within the magnetic resonance imaging data.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to an advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: head coil device
- 102: light source
- 104: light
- 106: target area
- 108: opening
- 110: rim
- 118: subject
- 120: head and neck region
- 122: head
- 124: support
- 130: lower part
- 132: upper part
- 134: reception
- 136: tuning circuit
- 150: medical system
- 152: MRI system
- 154: magnet
- 156: bore of magnet
- 157: inner surface of the bore
- 160: magnetic field gradient coils
- 161: radio-frequency antenna
- 162: gradient controller
- 163: radio-frequency transceiver
- 170: circuit
- 172: light emitting diode
- 174: resistor
- 180: optical sensor
- 300: computational system
- 302: computational component
- 304: memory
- 306: hardware interface
- 308: user interface
- 310: machine readable instructions
- 312: pulse sequence
- 314: MRI data
- 316: reconstructed MR images
- 318: optical sensor data
- 320: rPPG signal

## Claims

1. A head coil device (100) comprising one or more magnetic resonance imaging coil elements and at least one light source (102) for a remote photoplethysmography, the at least one light source (102) being configured to illuminate at least one target area (106), the illuminating being configured for providing light (104) emitted by the at least one light source (102) and scattered at the target area (106) for a remote photoplethysmography signal (320) acquisition from the at least one target area (106).

2. The head coil device (100) of claim 1, the at least one light source (102) being arranged at a rim (110) of an opening (108) of the head coil device (100), the opening (108) being configured for exposing the at least one target area (106).

3. The head coil device (100) of claim 2, the head coil device (100) being configured for receiving a head (122) of the subject (118) at a predefined position within the head coil device (100).

4. The head coil device (100) of claim 3, the opening (108) being configured for exposing at least part of a face of the head (122) received at the predefined position within the head coil device (100), the at least one target area (106) being comprised by one of the following sections of the exposed part of the face: a forehead, a cheek.

5. The head coil device (100) of claim 3, the opening (108) being configured for exposing at least part of a top region of the head (122) received at the predefined position within the head coil device (100), the at least one target area (106) being comprised by the exposed part of the top region of the head (122).

6. The head coil device (100) of claim 3, the opening (108) being configured for exposing at least part of a neck of the subject (118) with the head (122) received at the predefined position within the head coil device (100), the at least one target area (106) being comprised by the exposed part of the neck.

7. The head coil device (100) of any of the previous claims, the at least one light source (102) comprising at least two light emitting diodes (172).

8. The head coil device (100) of claim 7, the light emitting diodes (172) being arranged in an antiparallel circuit (170) integrated into a tuning circuit (136) of the head coil device (100) configured for tuning and detuning at least one coil element of the one or more of the coil elements.

9. The head coil device (100) of claim 8, the antiparallel circuit (170) comprising one or more resistors (174) configured for equalizing an intensity of the light emission by the light (104) emitting diodes during a tuning operation mode and a detuning operation mode of the tuning circuit (136).

10. The head coil device (100) of any of the previous claims, the at least one light source (102) being configured to emit one or more of the following: green light, infrared light, white light.

11. A medical system (150) comprising the head coil device (100) of any of the previous claims, the medical system (150) further comprises:
- a magnetic resonance imaging system (152) configured for acquiring magnetic resonance data from a subject (118) arranged at least partially with the head coil device (100) within a bore (156) of the magnetic resonance imaging system (152);
- at least one optical sensor (180) configured to acquire optical sensor data (318) comprising light (104) emitted by the at least one light source (102) of the head coil device (100), a field of view of the optical sensor (180) covering at least part of an inner surface (157) of the bore (156); and
- a computational system (300) comprising a memory (304) storing machine executable instructions (310) for controlling the medical system (150), wherein execution of the machine executable instructions (310) causes the computational system (300) to:
- emit light (104) by the at least one light source (102) for illuminating the at least one target area (106);
- receive the emitted light (104) scattered at the at least one target area (106) using the at least one optical sensor (180), the received light comprising emitted light (104), which is scattered additionally at the inner surface (157) of the bore (156);
- acquire a remote photoplethysmography signal (320) using the received light.

12. The medical system (150) of claim 11, the execution of the machine executable instructions (310) further causing the computational system (300) to use the acquired remote photoplethysmography signal (320) for a cardiac triggering of a magnetic resonance imaging data (314) acquisition executed using the magnetic resonance imaging system (152).

13. A method of operating a medical system (150), wherein the medical system (150) comprises:
- a head coil device (100) of any of the previous claims;
- a magnetic resonance imaging system (152) configured for acquiring magnetic resonance data from a subject (118) arranged at least partially with the head coil device (100) within a bore (156) of the magnetic resonance imaging system (152);
- at least one optical sensor (180) configured to acquire optical sensor data (318) comprising light (104) emitted by the at least one light source (102) of the head coil device (100), a field of view of the optical sensor (180) covering at least part of an inner surface (157) of the bore (156); and
- a computational system (300) comprising a memory (304) storing machine executable instructions (310) for controlling the medical system (150), wherein the method comprises:
- emitting light (104) by the at least one light source (102) for illuminating the at least one target area (106);
- receiving the emitted light (104) scattered at the at least one target area (106) using the at least one optical sensor (180), the received light comprising emitted light (104), which is scattered additionally at the inner surface (157) of the bore (156);
- acquiring a remote photoplethysmography signal (320) using the received light.

14. The method of claim 13, the method further comprising using the acquired remote photoplethysmography signal (320) for a cardiac triggering of a magnetic resonance imaging data (314) acquisition executed using the magnetic resonance imaging system (152).

15. A computer program comprising machine executable instructions (310) for execution by a computational system, wherein execution of the machine executable instructions (310) causes the computational system (300) to perform the method of any of claims 13 or 14.
